# EUROPEAN PATENT APPLICATION

(11) **EP 1 772 163 A1**
(43) Date of publication of application: **11.04.2007**
(21) Application number: 06110179.6
(22) Date of filing: 20.02.2006
(51) Int. Cl.: A61M 5/32, A61M 5/50

(54) **Safety syringe**

(71) Applicant: HSU, Chih-Kuang, West District Taichung City (TW)
(72) Inventor: HSU, Chih-Kuang, West District Taichung City (TW)
(74) Representative: Viering, Jentschura & Partner

(57) **Abstract**

The safety syringe comprises an injection cylinder (1) being a hollow cylinder with a receiving chamber (11) therein; a buckling seat (2) installed in the injection cylinder (1); the buckling seat (2) having a hard sleeve (21); a front end of the sleeve (21) being formed as a hollow post (211); a center of the buckling seat (2) being formed with a flow channel (212); a receiving space (215) being formed between an outer wall of the hollow post (211) and an inner wall of the sleeve (21); a needle unit (3) embedded into buckling seat (2); an outer side of the needle (32) being covered by a cover (33); the needle unit (3) being installed to a front end of the buckling seat (2); a tenon (41) resisting against the buckling portion (221); a push rod (4) inserted into and linearly moveable along the injection cylinder (1); and a seal (22) for enclosing a rear end of the sleeve (21) by molding injecting a rubber material.

## Description

### FIELD OF THE INVENTION

The present invention relates to syringes, and particularly to a safety syringe which makes the sleeve and seal can be tightly combined easily and safely. The sleeve is made of plastic and the seal is made of rubber.

### BACKGROUND OF THE INVENTION

The prior art safety syringe, Taiwan Patent No. 200503800, includes a buckling seat, a sleeve and a seal. The safety syringe serves to have a simple structure and to be assembled easily. In the prior art, the sleeve and the seal are formed directly formed. The sleeve has a hard outer cover and a rubber inner cover within the outer cover. A buckling portion extends from an inner side of the inner cover for resisting a tenon of a push rod so that when the push rod is pulled backwards, it will resist against the buckling portion. By above structure the sleeve and the seal are formed and the push rod and the buckling portion are resisted to one another. The needle will be pulled into the syringe cylinder without being reused.

However above mentioned prior art has a simple structure, but it the the following disadvantages. The outer cover is made of plastics and the inner cover is made of rubber. The combination of the inner cover and outer cover is not stable. Furthermore, only the inner side and the lower end of the inner cover are enclosed by the outer cover. The combination strength is low. Moreover, only the inner side of the inner cover is combined with the outer cover. The combination is weak. When the needle passes through the buckling portion of the inner cover, the inner cover is possibly reduced inwards. As a result, a gap is formed between the lower edges of the inner cover and the outer cover. Thus, the liquid in the injection cylinder will drain out. Further, it is difficult to assembly the syringe. Since the outer cover is made of PP material, if the injection cylinder is also made of PP material, the precisions of the two must be very high for having a preferred combination. However this will induce the high cost of the mold and the yield ratio is low.

However above mention defects still exist at many different kinds of prior art syringes, such as Taiwan Patent Nos. 259607, and Taiwan Patent No. 200503801.

### SUMMARY OF THE INVENTION

Accordingly, the primary object of the present invention is to provide a safety syringe which makes the sleeve and seal can be tightly combined. The sleeve is made of plastic and the seal is made of rubber.

To achieve above object, the present invention provides a safety syringe. The safety syringe comprises an injection cylinder being a hollow cylinder with a receiving chamber therein; a front end thereof being formed as a hollow tapered head; the tapered head having an internal hollow space which is communicated to the receiving chamber; the hollow space having a penetrating hole; an inner wall of the hollow space being installed with a stepped resisting portion; a buckling seat installed in the injection cylinder; the buckling seat having a hard sleeve; a front end of the sleeve being formed as a hollow post; a center of the buckle seat being formed with a flow channel which is communicated to the center hollow portion of the hollow post; a receiving space being formed between an outer wall of the hollow post and an inner wall of the sleeve; a concave chamber being formed between an outer wall of the post and the inner wall of the buckling seat for receiving a needle seat; a needle unit embedded into buckling seat; the needle unit having a needle seat and a needle secured to the needle seat; an outer side of the needle being covered by a cover; the needle unit being installed to a front end of the buckling seat; the tenon resisting against the buckling portion; after injection, the push rod can be pulled backwards; a push rod inserted into the push rod and linearly moveable along the injection cylinder; the push rod having a tenon at a front end thereof; and a seal for enclosing a rear end of the sleeve by molding injecting a rubber material; the seal also enclosing the inner wall of the receiving space so as to increase the tightness between the seal and the sleeve.

The various objects and advantages of the present invention will be more readily understood from the following detailed description when read in conjunction with the appended drawing.

### BRIEF DESCRIPTION OF THE DRAWINGS

Figs. 1 is an exploded schematic view of the present invention.
Fig. 2 is a schematic cross view of the present invention.
Fig. 3 is a partial enlarged view of Fig. 2.
Fig. 4 is a schematic cross view about the operation of the present invention.
Fig. 5 is a schematic cross view about another operation of the present invention.
Fig. 6 is a schematic perspective view of the second embodiment of the present invention.
Fig. 7 is a cross sectional view about the operation of the second embodiment of the present invention.

### DETAILED DESCRIPTION OF THE INVENTION

In order that those skilled in the art can further understand the present invention, a description will be described in the following in details. However, these descriptions and the appended drawings are only used to cause those skilled in the art to understand the objects, features, and characteristics of the present invention, but not to be used to confine the scope and spirit of the present invention defined in the appended claims.

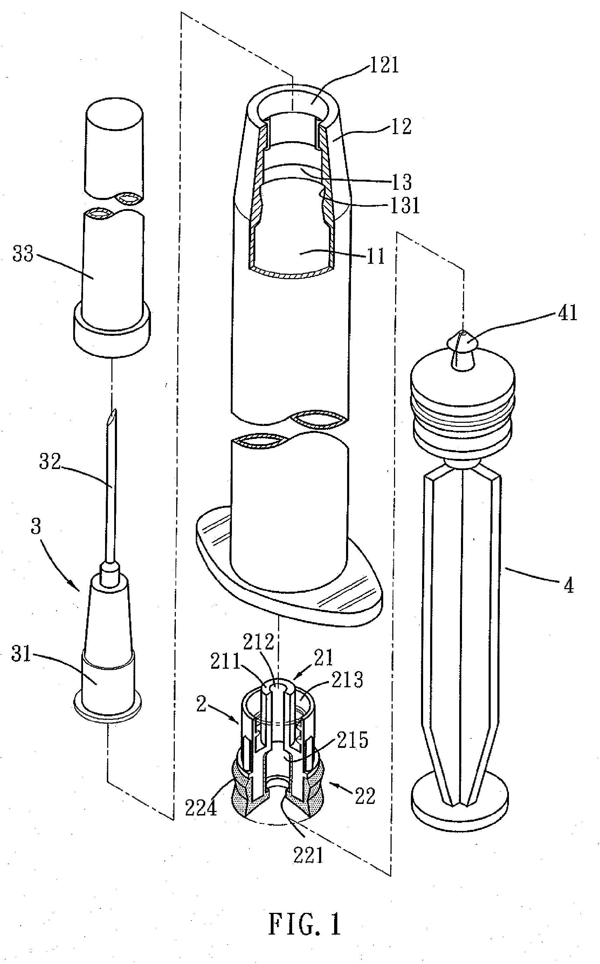

Referring to Fig. 1, the safety syringe of the present invention is illustrated. The present invention has the following elements.

An injection cylinder 1 is a hollow cylinder with a receiving chamber 11 therein. A front end thereof is formed as a hollow tapered head 12. The tapered portion 12 has an internal hollow space 13 which is communicated to the receiving chamber 11. The hollow space 13 has a penetrating hole 121. An inner wall of the hollow space 13 is installed with a stepped resisting portion 131.

A buckling seat 2 is installed in the injection cylinder 1. The buckling seat 2 has a hard sleeve 21. A front end of the sleeve 21 is formed as a hollow post 21. A center of the buckle seat 2 is formed with a flow channel 212 which is communicated to the center hollow portion of the hollow post 21. A receiving space 215- is formed between an outer wall of the hollow post 211 and an inner wall of the sleeve 21. A concave chamber 213 is formed between an outer wall of the post 211 and the inner wall of the buckling seat 2 for receiving a needle seat 31.

A needle unit 3 is embedded into buckling seat 2. The needle unit 3 has a needle seat 31 and a needle 32 secured to the needle seat 31. An outer side of the needle 32 is covered by a cover 33. The needle unit 3 is installed to a front end of the buckling seat 2. The tenon 41 resists against the buckling portion 221. After injection, the push rod 4 can be pulled backwards.

A push rod 4 is inserted into the push rod 4 and is linearly moveable along the injection cylinder 1. The push rod 4 has a tenon 41 at a front end thereof.

A seal 22 serves to enclose a rear end of the sleeve 21 by molding injecting a rubber material. The seal 21 also encloses the inner wall of the receiving space 215 so as to increase the tightness between the seal 21 and the sleeve 21.

A bottom end of the seal 22 is protruded inwards with a buckling portion 221. The buckling portion 221 can be buckled to the tenon 41 of the push rod 4. Thereby after injection, the push rod 4 can be pulled along a reverse direction, and then the buckling seat 2 will be driven into the receiving chamber 11 of the injection cylinder 1. An outer wall of the seal 22 will resist against a ring 224 at an outer wall of the seal 22. The ring 24 is corresponding to a resisting portion 13 i at an inner hollow space of the tapered head 12.

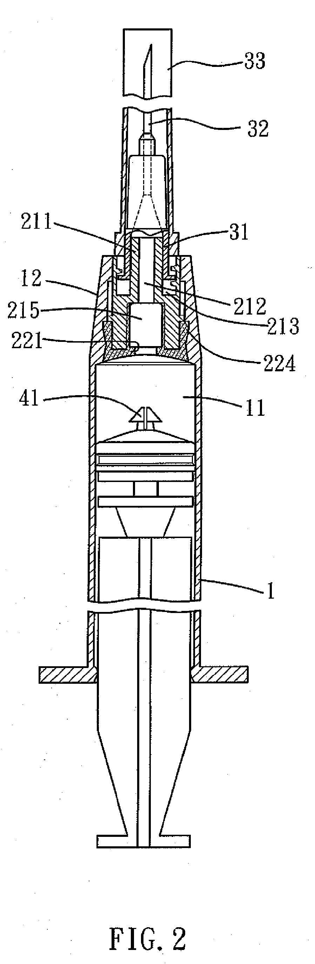

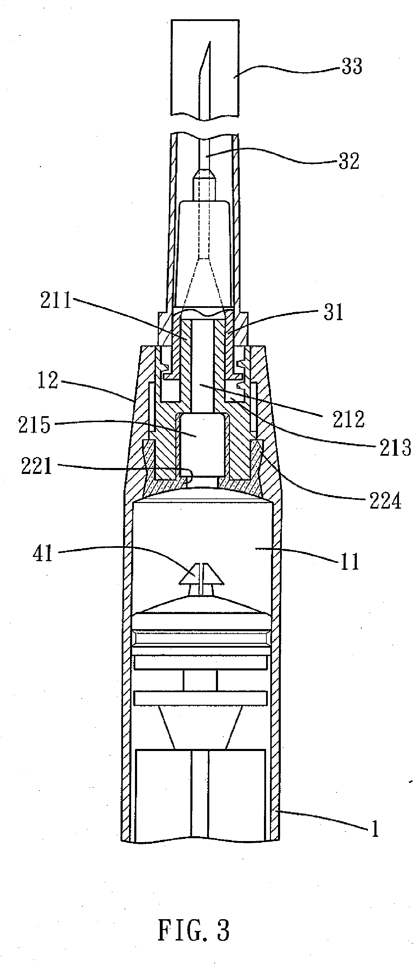

Referring to Figs. 2 and 3, in assembly, the needle seat 31 of the needle unit 3 is engaged to the concave chamber 213 of the sleeve 21. Then the buckling seat 2 is inserted into an opening at a lower side of the receiving chamber 11 of the injection cylinder 1 so that a front end of the sleeve 21 is tightly engaged to the penetrating hole 121 of the tapered portion 12. A rear end of the sleeve 21 after the hollow space 13 is enclosed by the seal 22. The ring 224 at the seal 22 tightly resists against the resisting portion 131 of the tapered head 12. Thereby the liquid within the receiving chamber 11 will not drain out in the injection. Then the push rod 4 is placed into the opening of the receiving chamber 11.

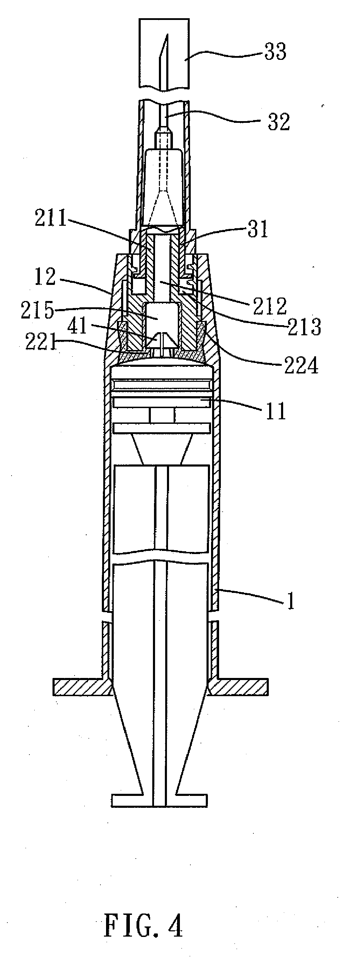

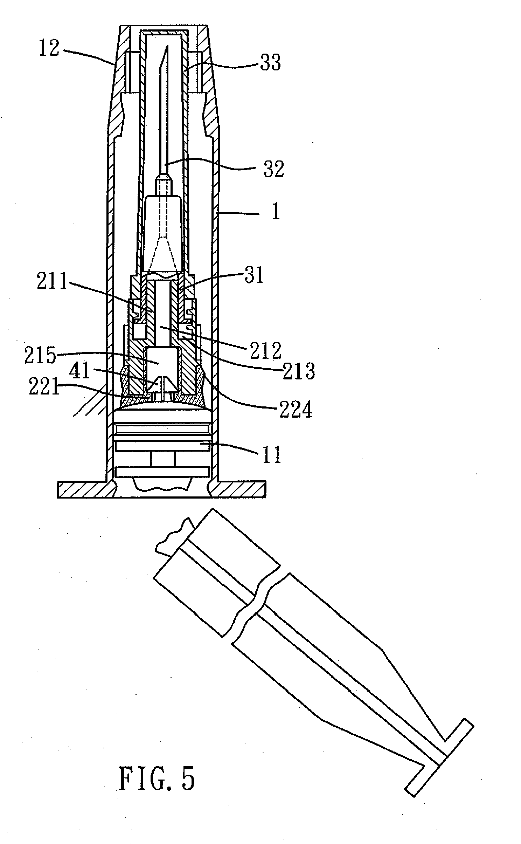

Referring to Figs. 4 and 5, in use, after injection, the tenon 41 resists against the buckling portion 221. When the push rod 4 is pulled backwards, the needle unit 3 will be pulled to reduce into the receiving chamber 11 of the injection cylinder 1. Then a lateral pressure is applied to break the push rod 4. The needle unit 3 and the buckling seat 2 are remained in the injection cylinder 1. Thereby the syringe can no be reused.

Besides, since the sleeve 21 of the buckling seat 2 is sealed by a rubber seal. When the buckling seat 2 is engaged to the tapered head 12 of the injection cylinder 1, the deformation of the rubber seal will make the rubber sleeve 21 being tightly engaged to the rubber seal.

Furthermore, the ring of the rubber seal has the effect of the prior art O ring. The 0 ring and the seal are integrally formed. Thereby the assembly of the 0 ring is reduced. The buckling seat 2 is preferably sealed to the tapered head 12.

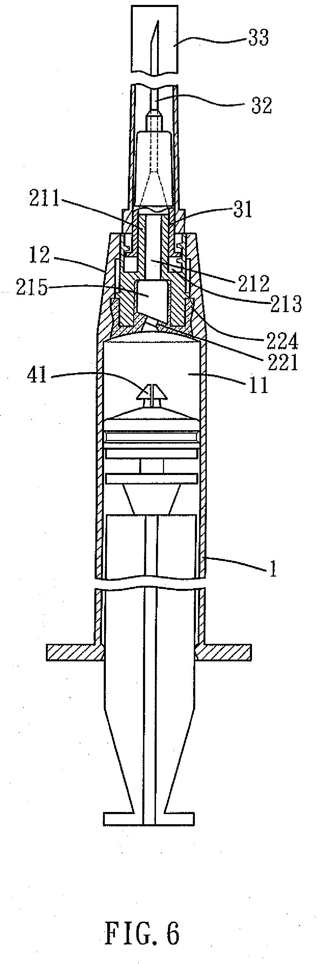

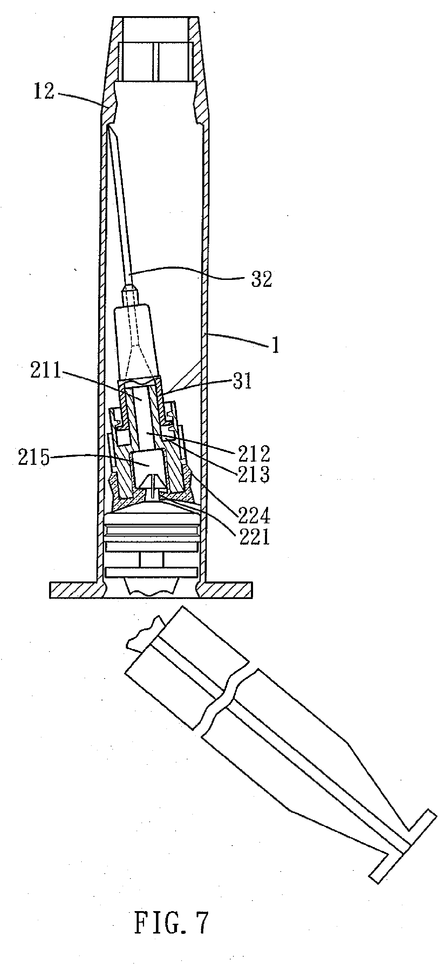

Referring to Figs. 6, 7, another embodiment of the present invention is illustrated. Those identical from above mentioned will be numerated with the same numerals, only those difference between the two are described. In this the present invention, an upper surface of the buckling portion 22 has an inclined surface. When the needle unit 3 is broken, it will be inclinedly arranged.

The present invention is thus described, it will be obvious that the same may be varied in many ways. Such variations are not to be regarded as a departure from the spirit and scope of the present invention, and all such modifications as would be obvious to one skilled in the art are intended to be included within the scope of the following claims.

## Claims

1. A safety syringe comprising:
an injection cylinder being a hollow cylinder with a receiving chamber therein; a front end thereof being formed as a hollow tapered head; the tapered head having an internal hollow space which is communicated to the receiving chamber; the hollow space having a penetrating hole; an inner wall of the hollow space being installed with a stepped resisting portion;
a buckling seat installed in the injection cylinder; the buckling seat having a hard sleeve; a front end of the sleeve being formed as a hollow post; a center of the buckle seat being formed with a flow channel which is communicated to the central hollow portion of the hollow post; a receiving space being formed between an outer wall of the hollow post and an inner wall of the sleeve; a concave chamber being formed between an outer wall of the post and the inner wall of the buckling seat for receiving a needle seat;
a needle unit embedded into buckling seat; the needle unit having a needle seat and a needle secured to the needle seat; an outer side of the needle being covered by a cover; the needle unit being installed to a front end of the buckling seat;
a push rod inserted into the push rod and linearly moveable along the injection cylinder; the push rod having a tenon at a front end thereof; the tenon resisting against the buckling portion; after injection, the push rod can be pulled backwards; and
a seal for enclosing a rear end of the sleeve by molding injecting; the seal also enclosing the inner wall of the receiving space so as to increase the tightness between the seal and the sleeve.

2. The safety syringe as claimed in claim 1, wherein a bottom end of the seal is protruded inwards with a buckling portion; the buckling portion is buckled to the tenon of the push rod; thereby after injection, the push rod can be pulled along a reverse direction, and then the buckling seat will be driven into the receiving chamber of the injection cylinder; an outer wall of the seal will resist against a ring at an outer wall of the seal; the ring is corresponding to a resisting portion at an inner hollow space of the tapered head.

3. The safety syringe as claimed in claim 1, wherein an upper surface of the buckling portion has an inclined surface.

4. The safety syringe as claimed in claim 1, wherein the sleeve is made of plastics and the seal is made of rubber.
